# EUROPÄISCHE PATENTSCHRIFT

(11) **EP 4 031 233 B1**
(45) Veröffentlichungstag und Bekanntmachung des Hinweises auf die Patenterteilung: **24.12.2025**
(21) Anmeldenummer: 20767750.1
(22) Anmeldetag: 02.09.2020
(51) Int. Cl.: A61N 1/36, A61N 1/04, A61B 5/026, A61B 5/145

(54) **SYSTEM ZUR LOKALEN AKTIVIERUNG DES MENSCHLICHEN AUGES UND DES GEHIRNS ZUM SEHLEISTUNGSTRAINING, INSBESONDERE ZUR AKTIVIERUNG DES VISUELLEN KORTEXES UND REORGANISATION NEURONALER NETZWERKE IM MENSCHLICHEN GEHIRN FÜR EINE STÄRKUNG DES RESIDUALSEHENS**
SYSTEM FOR LOCALLY ACTIVATING THE HUMAN EYE AND THE BRAIN IN ORDER TO TRAIN VISUAL PERFORMANCE, IN PARTICULAR IN ORDER TO ACTIVATE THE VISUAL CORTEX AND REORGANIZED NEURAL NETWORKS IN THE HUMAN BRAIN IN ORDER TO STRENGTHEN RESIDUAL VISION
SYSTÈME POUR ACTIVER LOCALEMENT L'OEIL HUMAIN ET LE CERVEAU DE FAÇON À ENTRAÎNER DES PERFORMANCES VISUELLES, EN PARTICULIER DE FAÇON À ACTIVER LE CORTEX VISUEL ET LES RÉSEAUX NEURONAUX RÉORGANISÉS DANS LE CERVEAU HUMAIN DE FAÇON À RENFORCER LA VISION RÉSIDUELLE

(30) Priorität: 20.09.2019 DE 102019125416; 27.09.2019 DE 102019126163; 09.10.2019 DE 102019127098; 11.11.2019 DE 102019130302
(43) Veröffentlichungstag der Anmeldung: 27.07.2022
(73) Patentinhaber: Savir GmbH, 14163 Berlin (DE)
(72) Erfinder: GAO, Ying, 39106 Magdeburg (DE); SABEL, Kornelia, 14163 Berlin (DE)
(74) Vertreter: Meissner Bolte Partnerschaft mbB
(86) Internationale Anmeldenummer: PCT/EP2020/074408
(87) Internationale Veröffentlichungsnummer: WO 2021/052754

(56) Entgegenhaltungen:
- EP-A1- 3 598 962
- WO-A1-2013/037618
- WO-A1-2014/141213
- WO-A1-2017/048731
- WO-A1-2017/222997
- CN-A- 109 045 435
- CAROLIN GALL ET AL: "Alternating Current Stimulation for Vision Restoration after Optic Nerve Damage: A Randomized Clinical Trial", PLOS ONE, vol. 11, no. 6, 29 June 2016 (2016-06-29), pages e0156134, XP055746806, DOI: 10.1371/journal.pone.0156134

## Beschreibung

Die Erfindung betrifft ein System zur lokalen Aktivierung des menschlichen Auges und des Gehirns zum Sehleistungstraining, insbesondere zur Aktivierung von Nervenzellen im menschlichen Auge und Gehirn für eine Stärkung des Residualsehens bei bestehenden Gesichtsfelddefekten mittels nicht invasiver Impulsbehandlung, insbesondere Wechselstromimpulsbehandlung (tASC), bestehend aus einem Applikator zum Führen eines Stromflusses zum Auge und/oder zum Gehirn und zur Anregung der Durchblutung und Aktivierung von Nervenzellen des Sehsystems, insbesondere retinaler Ganglienzellen, einen Impulsgenerator zur Erzeugung von elektrischen Stimulationssignalen sowie einer Datenverarbeitungs- und Steuereinheit zum Bereitstellen patientenspezifischer Stimulationssignalfolgen, wobei der Applikator mindestens zwei Elektroden aufweist, welche am Kopf des Probanden zur Anlage bringbar sind, gemäß Oberbegriff des Anspruches 1.

Gesichtsfelddefekte gelten bisher als irreversibel, da sich Nervenzellen der Retina, des Nervus opticus und des Gehirns nicht regenerieren können. Es hat sich jedoch gezeigt, dass eine Teilerholung von Gesichtsfelddefekten möglich ist, da das Gehirn, welches retinale Signale auswertet und interpretiert, die residualen Signale über bestimmte Mechanismen dauerhaft verstärken kann. Ebenso finden sich in der Retinal Nervenzellen, die nach einem pathologischen Ereignis ihre Funktion reduzieren und "erstummen", da sie keine Nervensignale mehr aussenden können, ohne aber abzusterben.

Da die Erfahrung zeigt, dass bei fast allen Patienten noch Restsehleistungen, das heißt ein sogenanntes Residualsehen, vorhanden sind, wurde vorgeschlagen, eine Reaktivierung der "stummen" Nervenzellen vorzunehmen und über die Verbesserung synaptischer Übertragung die Restsehleistungen zu stärken. Klinische Studien zeigen den Erfolg von Behandlungsverfahren des Sehtrainings oder der Wechselstromstimulation. Bei der Wechselstrombehandlung wird die gesamte Retina und Teile des Gehirns aktiviert und synchronisiert. Ein weit überwiegender Teil der Patienten spricht durch oben erwähnte Therapieformen gut an. Physiologische Untersuchungen der Mechanismen von Wechselstrom mit EEG und fMRT weisen auf eine massive lokale und globale Änderung der Durchblutung und der Reorganisation neuronaler Netzwerk im Gehirn hin. Eine lokale Aktivierung der Nervenzellen findet in den Augen und im visuellen System des Gehirns über eine globale Reorganisation neuronaler Netzwerke statt. Da über die Reaktivierung von Nervenzellen und eine Modulation der Neuroplastizität das Residualsehen gestärkt werden kann, kommt nicht nur den Augen sondern auch dem Gehirn für die visuelle Rehabilitation in der Augenheilkunde eine große Bedeutung zu.

Seit einiger Zeit wird die Anpassungsfähigkeit des Gehirns, auch Plastizität genannt, für die Ophthalmologie genutzt. Über Methoden der Neuromodulation mittels schwacher Wechselströme können teilgeschädigte visuelle Funktionen über den Prozess der Neuroplastizität gestärkt werden.

In jüngerer Zeit wurden Verfahren zur Durchblutungsverbesserung und Resynchronisation der Netzwerkaktivität des Gehirns mittels Wechselstromimpulsen erprobt und bei teilerblindeten Patienten getestet. Hierbei wird Wechselstrom mit Hilfe spezieller Elektroden an der Stirn nicht invasiv für mehrere Tage in definierten Sitzungen verabreicht. Der Wechselstrom fließt hierbei von den Elektroden zum Auge und regt dort retinale Ganglienzellen dazu an, in vorbestimmten Frequenzen zu feuern mit dem Ziel der Resynchronisation von Hirnnetzwerken. Eine Schädigung des Nervus opticus führt nämlich zu einer Desorganisation funktioneller Netzwerke im Gehirn, was sich durch eine neurophysiologische Desynchronisation im EEG bemerkbar macht. Behandlungen mittels Wechselstrom können diesen Zustand über eine Durchblutungsverbesserung und Resynchronisation von Hirnnetzwerkaktivität deutlich verbessern.

Die Resynchronisation der Sehleistung ist jedoch individuell sehr unterschiedlich und hängt von den unterschiedlichsten Faktoren ab.

Dabei kommt es neben einer Optimierung der Stimulationsimpulse auch darauf an, den Patienten zumindest während der Behandlung weitgehend stressfrei zu halten. Hierfür besteht die Notwendigkeit der Schaffung einer optimierten Ausbildung von Stimulationselektroden einschließlich technischer Mittel zur Halterung dieser Elektroden am Patientenkopf. Es muss also ein Applikator geschaffen werden, der sowohl ergonomisch optimal als auch behandlungstechnisch besonders geeignet ist und allen Anforderungen genügt.

Wesentlich ist darüber hinaus, eine Optimierung der Stimulation so auszugestalten, dass ein Behandlungserfolg auch über die Stimulationszeit hinaus und möglichst lange aufrechterhalten bleibt.

Zum Stand der Technik sei im Hinblick auf die vorgenannten Aspekte auf die EP 1 603 633 B1 verwiesen, die ein spezielles Befestigungsteil für eine Stimulationsvorrichtung an einer gewünschten Position auf dem Kopf des Patienten zeigt, wobei eine Einstellungsstruktur aus einem verformbaren Werkstoff hergestellt ist, die dazu in der Lage ist, die Vorrichtung an Umrisse des Kopfes des Patienten anzupassen.

Befestigungsvorrichtungen oder Anordnungen zur Kontaktanlage von Stimulationselektroden sind darüber hinaus in den EP 1 708 787 und EP 1 734 877 B1 offenbart.

Bezogen auf den Stand der Technik hinsichtlich der Hirnstimulation mit zusätzlicher Kontrolle des Durchblutungsverhaltens benachbarter Gefäße sei noch auf die WO 2011/106660 A1 und die WO 2016/115392 A1 aufmerksam gemacht.

Bei der WO 2011/106660 A1 wird auf das Erkennen der Wirkung einer Stimulation und insbesondere das gleichzeitige Stimulieren und Erfassen der Stimulationswirkung in Echtzeit abgestellt. Hierzu wird während der Stimulation auf nicht-invasiver Weise eine funktionelle Nahinfrarotspektroskopie genutzt. Dabei wird die funktionelle Nahinfrarotspektroskopie verwendet, um den Oxygenerierungszustand von Hämoglobin zu messen.

Die WO 2016/115392 A1 bezieht sich auf Vorrichtungen und Verfahren zum Bestimmen der neurovaskulären Reaktivität unter Verwendung einer Stimulation des neurovaskulären Systems und gleichzeitiger Aufzeichnung einer neuronalen hämodynamischen Antwort darauf.

Hinsichtlich der verschiedenen Möglichkeiten der Ausbildung von Stimulationselektroden und entsprechender Applikatoren sei auf den nachfolgend kurz umrissenen Stand der Technik aufmerksam gemacht.

Die US 5 522 864 A offenbart eine stirnbandähnliche Elektrodenhalterung. Eine erste Elektrode wird auf das geschlossene Augenlid eines Patienten aufgebracht. Die zweite Elektrode ist im Stirnband fixiert. Eine dritte Elektrode im Nackenbereich angebracht. Eine derartige Elektrodenausbildung, insbesondere das Anbringen einer Elektrode auf einem geschlossenen Augenlid, wird vom Patienten als unangenehm empfunden und versetzt ihn in einen, für den Behandlungserfolg negativen Stress.

Auch bei der Patentfamilie um die EP 30 13 414 B1, die eine Stimulationsvorrichtung für eine transdermale elektrische Stimulation zeigt, sind mehrere Elektroden an voneinander deutlich entfernten Positionen fixiert. Eine erste Elektrode wird am Stirnbereich des Patienten befestigt. Eine zweite Elektrode befindet sich am Nacken oder der Schulter des Patienten. Diese Elektrodenanordnung ist schlecht individualisierbar und kann vom Patienten nur mit Hilfe Dritter optimal positioniert werden, was für eine Heimtherapie einen erheblichen Nachteil darstellt.

Bei der EP 33 49 844 A1 geht es um die Behandlung durch Anwendung von Mikroströmen. Diesbezüglich werden punktuelle Stimulationselektroden oberhalb und unterhalb des Auges im Lidbereich angebracht. Eine derartige Positionierung unmittelbar im empfindlichen Augenbereich ist für eine Vielzahl der Patienten unangenehm, insbesondere auch deshalb, weil die Elektroden relativ klein sind und somit keine ausreichende Stromstärke ermöglichen. Bereits sehr geringe Ströme sind spürbar, die aber therapeutisch nicht ausreichend wirksam sind. Gerade im Bereich oberhalb und unterhalb des Auges ist die menschliche Haut sehr empfindlich für Reizungen, so dass eine derartige Elektrodenausbildung keine nennenswerte Verbreitung fand.

Die EP 2 981 326 B1 zeigt ein Head-Set-ähnliche Anordnung zur Elektrostimulation der Hautoberfläche des Kopfes.

Einen brillenartigen Träger für Stimulationselektroden offenbart die DE 10 2011 055 844 B4, wobei zusätzlich eine Nasenabstützung, ähnlich einer typischen Brille vorhanden ist. Die Elektroden sind dort abnehmbar, das heißt austauschbar, ausgeführt. Allerdings werden bei dieser Anordnung Haarelektroden eingesetzt, die direkt auf der Cornea des Augapfels liegen, was Risken der Corneaschädigung mit sich bringt.

Bei der EP 2 651 504 B1 ist ein Stirnband als Elektrodenträger zur Neurostimulation vorgesehen, wobei das Stirnband im Kopfbereich des Patienten zur Anlage kommt und diesbezügliche Elektroden symmetrisch am Hinterkopf des Patienten zur Anlage kommen. Weiterhin sind elektrische Anschlüsse zur Verbindung der Elektroden mit einem separaten Impulsgenerator vorhanden.

Bei der Vorrichtung nach der US 2006/129207 A zur elektrischen Stimulation von Ganglienzellen wird wiederum von einem brillenähnlichen Gebilde ausgegangen, das Elektroden besitzt, die mit dem Lid des Probanden in Verbindung gebracht werden. Über eine Abstützung soll der sonst unangenehme Druck auf die Augenlider minimiert werden, woraus jedoch der Nachteil einer nicht immer eindeutig reproduzierbaren Größe des Übergangswiderstandes zwischen den Elektroden und der Haut des Probanden resultiert.

Aus der CN 109045435 A ist eine Vorrichtung zum Stimulieren des visuellen Kortex beim Menschen vorbekannt. Eine Elektrodenanordnung liegt an beiden Schläfen bzw. im Bereich zwischen Schläfe und Wange an. Die Stimulation soll die Nerven rund um das Auge anregen, die Augenmuskeln relaxen und die Blutzirkulation um die Augen verbessern. Primär ist die dortige Lehre auf eine musische Stimulation des visuellen Kortex gerichtet, wobei eine elektrische Stimulation nur zusätzlich wirkt und hierbei Stromstärken zwischen 0 und 10 mA Anwendung finden. Mittels einer Optik und Augenmuscheln am Stimulationsgerät kann der Proband bei diesem Stand der Technik auf einem Display bewegliche Szenen erkennen, d.h. der Proband unterliegt zusätzlichen optischen Reizen.

Aus der WO 2013/037618 A1 sind Stimulationselektroden mit den bereits beschriebenen Nachteilen vorbekannt.

Nachteilig ist auch die Anordnung von Elektroden im Lidbereich, d.h. oberhalb und unterhalb des menschlichen Auges, wie in der WO 2017/048731 A1 gezeigt.

Bekannt ist darüber hinaus auch eine optische Stimulierung, wie in der WO 2017/222997 A1 beschrieben.

Zusammenfassend zeigt der Stand der Technik eine Vielzahl unterschiedlicher Applikatoren zur Elektrostimulation auch für den hier relevanten Fall der lokalen Aktivierung der Augen und des Gehirns, wobei die Mehrzahl der bekannten Applikatoren für eine Heimtherapie, das heißt im Sinne einer reproduzierbaren, einfachen, und risikofreien Anwendung durch den Patienten ohne medizinische oder ärztliche Unterstützung nicht geeignet ist.

Aus diesem Grunde - und letztendlich auch aus Kostenerwägungen - wird derzeit weit überwiegend auf großflächige Klebe-Stimulationselektroden zurückgegriffen, die nach Erfahrungswerten des medizinischen Personals am jeweiligen Probanden oder Patienten angebracht werden. Der Vorteil größerer Elektroden liegt darin, dass eine ausreichende Stromstärke verabreicht werden kann, da der Stromfluß über eine größere Fläche in die Haut, Augen, oder Schädel eindringt. Diesbezüglich wird häufig eine Trialand-Error-Methode genutzt mit der Folge einer nur sehr eingeschränkten Reproduzierbarkeit. Heimanwendungen sind gerade auch auf der Basis von Klebeelektroden nahezu ausgeschlossen, so dass grundsätzlich eine ambulante Vorbereitung der Therapie erforderlich ist.

Der Stand der Technik berichtet also von sehr unterschiedlichen Elektroden-größen und Positionierungen, die aber in ihrer Wirkung sehr variabel und damit nicht ausreichend reproduzierbar sind. Darum ist es wünschenswert, eine geeignete Elektrodenplatzierung zu ermöglich, die auch bei geringeren Strömen und auch mit geringerer Variabilität das Sehsystem modulieren kann.

Aus dem Vorgenannten ist es Aufgabe der Erfindung, eine individuelle Elektrodenanpassung anhand der patientenkonkreten physischen Voraussetzungen vorzunehmen. Hierfür soll ein neuartiges System geschaffen werden, das einerseits eine optimale Anordnung der Stimulationselektroden schafft und das eine anhaltende Wirkung nach der Stimulationsbehandlung und nicht nur während dieser sicherstellt.

Die Lösung der Aufgabe der Erfindung erfolgt mit einem System gemäß der Merkmalskombination nach Anspruch 1, wobei die Unteransprüche zweckmäßige Ausgestaltungen und Weiterbildungen umfassen.

Dabei ist es ein wesentlicher Aspekt der Erfindung, die Anwendung des Applikators intuitiv zu gestatten, wobei über eine Anordnung zur nicht-invasiven Bestimmung der Durchblutung der Haut und des Gehirns des Probanden und zur Bestimmung der Sauerstoffsättigung eine optimale Stimulationsfolge findbar ist, die zu einer maximalen Durchblutungssteigerung und zu einem am längsten anhaltenden Nacheffekt führt, derart, dass auch nach der Stimulation die Blutflusssteigerung und neuronale Aktivierung möglichst lange anhält, was ein deutlicher Indikator für den angestrebten Behandlungserfolg ist.

Der Impulsgenerator zur Erzeugung der Stimulationssignalfolgen kann ein Ein- oder Mehrkanalstimulator sein, der mit einer Datenverarbeitungs- und Steuerungseinheit zusammenwirkt, so dass patientenspezifische Stimulationssignalfolgen erzeugt werden.

Die Schläfen-Positionierung ermöglich es, beide Augen gleichzeitig mit nur einem Kanal zu stimulieren, was die Kosten der Herstellung des Stimulationsgenerators reduziert und die Effektivität und Zuverlässigkeit, d. h. eine geringere Variabilität des Erfolgs verbessert.

Die optimalen Stimulationssignalfolgen können bei einer Erstbehandlung unter ärztlicher Aufsicht ermittelt, dann abgespeichert und dem Patienten auf einer Patientenspeicherkarte zur Heimanwendung übergeben werden. Ebenso ist es möglich, eine Datenspeicherung unmittelbar in einer Speichereinheit des Applikators vorzunehmen.

Der Applikator ist so aufgebaut, dass nach einer kurzen Erläuterung der Handhabung ein intuitiver Einsatz, insbesondere auch im Heimbereich erfolgen kann.

Der Applikator umfasst also einen Elektrodenhalter, der insbesondere individuell an die Kopfform anpassbar ist. Weiterhin erfolgt eine Schläfenpositionierung der Elektroden für eine optimale Stimulation. Hieraus ergibt sich der Vorteil, dass die Stromstärke der Impulsfolgen reduziert werden kann und trotzdem ausreichend Phosphene entstehen. Über ein NIR-Feedbacksystem kann eine Maximierung des hämodynamischen Nacheffektes bei der nicht-invasiven Augen- und Hirnsimulation geschaffen werden.

Im Zuge umfangreicher Untersuchungen und Studien, zurückgehend auf die Anmelderin, hat sich gezeigt, dass die Anordnung der Elektroden eine maßgebliche Rolle für den Stimulationserfolg des Sehsystems innehat.

Diesbezüglich wird eine sogenannte F7-F8 Anordnung gemäß der Nomenklatur des EEG 10-20 Systems im Bereich der Schläfen des Patienten vorgeschlagen. Bei einer solchen Anordnung werden Phosphene registriert, und zwar mit äußerst geringen Stromstärken im Bereich kleiner 2 mA bei einer Wechselstromfrequenz im Bereich von 8-25 Hz. Werden Elektroden im F7-F8 Bereich positioniert, kann der Patient die Augen schließen oder diese in einem dunklen Raum offenhalten. Ein unangenehmer Druck im Bereich des Lides bzw. ober- oder unterhalb des Auges durch angesetzte oder angelegte Elektroden tritt nicht auf.

Nutzt man Nasselektroden und ordnet diese im Bereich F7-F8 an, tritt darüber hinaus ein sehr angenehmer Kühleffekt auf, welcher zum Stressabbau führt, den Hautwiderstand verringert und ebenso den Behandlungserfolg verbessert.

Obiges vorangestellt, betrifft die Erfindung ein System zur lokalen Aktivierung der menschlichen Augen und des Gehirns zur Reorganisation neuronaler Netzwerke für eine Stärkung des Residualsehens und Durchblutungsverbesserung bei bestehenden Gesichtsfelddefekten. Diesbezüglich kommt eine Impulsstrombehandlung, insbesondere nicht-invasive Wechselstromimpulsbehandlung, zum Einsatz.

Das System besteht aus einem Applikator zum Führen des Stromflusses zum Auge und Gehirn und zur Anregung der Durchblutung und Aktivierung von Nervenzellen, insbesondere retinaler Ganglienzellen.

Das System umfasst darüber hinaus einen Impulsgenerator zur Erzeugung von elektrischen Stimulationssignalen sowie eine Datenverarbeitungs- und Steuereinheit zum Bereitstellen patientenspezifischer Stimulationssignalfolgen.

Der Applikator weist mindestens zwei Elektroden auf, die am Kopf des Probanden in der noch erläuterten Weise zur Anlage bringbar sind.

Am Applikator sind austauschbare Stimulationselektroden so fixiert, dass diese rechts und links vom Auge im Bereich der Schläfe des Patientenkopfes auf- oder anliegen, das heißt im sogenannten F7-F8 Bereich.

Weiterhin weist der Applikator erfindungsgemäß mindestens eine Anordnung zur nicht-invasiven Bestimmung der Durchblutung der Haut und des Gehirns und zur Bestimmung der Sauerstoffsättigung, insbesondere auf der Basis der fNIR-Spektroskopie auf.

Ein NIR-Emitter und der mindestens eine zugehörige NIR-Detektor sind in entfernter Position von den Stimulationselektroden am Kopf des Patienten und separat von den Stimulationselektroden positioniert.

Die NIR-Spektroskopiedaten, insbesondere in Pausen oder am Ende von Stimulationssignalfolgen, werden erfasst, um die Zeitdauer einer anhaltenden, verbesserten Durchblutung und/oder Sauerstoffsättigung zu bestimmen, so dass hiernach über das erfindungsgemäße System ein aktualisiertes Betreiben der Datenverarbeitungs- und Steuereinheit erfolgen kann.

In Weiterbildung der Erfindung ist der Applikator individuell an die Kopfform des Patienten anpassbar. Hierfür sind an sich bekannte Verstellmittel hinsichtlich der Fixierung am Stirn- und Hinterkopfbereich des Patienten ausgebildet.

In einer Ausgestaltung der Erfindung ist der Applikator als am Kopf anlegbares ring- oder kronenförmiges Gebildet ausgeführt, wobei eine Nasenrückenabstützung und/oder eine Ohrabstützung und/oder eine Hinterkopfabstützung vorgesehen ist.

Darüber hinaus kann im Hinterkopfbereich eine Aufnahme für elektronische Komponenten und zum Gewichtsausgleich vorgesehen werden. Die elektronischen Komponenten können hier eine Batterie oder einen Akkumulator umfassen, so dass der Applikator frei von externen Verkabelungen zur Stromversorgung genutzt werden.

In einer Ausgestaltung der Erfindung sind die Stimulationselektroden als Trocken- oder Nass-Kissenelektrode ausgebildet.

Bei einer Nass-Kissenelektrode kann als Feuchtigkeitslösung ein Elektrolyt zur Anwendung kommen, um den Übergangswiderstand zwischen der jeweiligen leitfähigen Komponente in der Elektrode und der Oberfläche der Haut des Patienten zu reduzieren. Je geringer der Übergangswiderstand ist, je niedriger kann der Strom für die Impulsfolgen gewählt werden und es kommt nicht zu einem unangenehmen Effekt im Sinne eines typischen Kribbelns bei gefühltem elektrischen Stromfluss. Am Applikator können Fixierpunkte zum Anbringen, insbesondere Anclipsen, weiterer Elektroden vorgesehen sein, welche eine Strombeaufschlagung, insbesondere Wechselstrombeaufschlagung auch im Bereich unterhalb der Augen des Patienten ermöglichen, wenn dies aus therapeutischen Gesichtspunkten von Vorteil ist.

Am oder im Applikator ist mindestens eine Schnittstelle zur drahtgebundenen oder drahtlosen Datenübertragung ausgebildet.

Der Applikator kann auf diese Weise mit einem übergeordneten System über eine sogenannte Luftschnittstelle Informationen austauschen, den Verlauf der Behandlungsdauer und den Behandlungserfolg registrieren und diese Daten dem behandelnden Arzt zur Auswertung und weiteren Optimierung der Behandlung zur Verfügung stellen bzw. übermitteln.

In einer Ausgestaltung der Erfindung sind am Applikator Schallerzeugungsmittel, insbesondere ausgebildet als Ohr- oder Kopfhörer, vorgesehen, um dem Patienten während der Behandlung durch Informationen, insbesondere zur Handhabung des Applikators zu führen und/oder akustisch zu entspannen.

Für den autarken Betrieb umfasst der Applikator sowohl den Impulsgenerator, insbesondere den Wechselstromimpulsgenerator als auch die Datenverarbeitungs- und Steuereinheit nebst Stromversorgung, wobei der Behandlungsablauf und/oder die Stimulationssignalfolgen durch ein Computerprogrammprodukt realisierbar sind. Insofern besitzt also der Applikator alle für den Einsatz und die Behandlung notwendigen technischen Mittel. Es ist demnach nicht mehr zwingend, eine separate Impulserzeugungseinheit vorzusehen, die entweder drahtgebunden neben dem Patienten steht oder vom Patienten mitzuführen ist, zum Beispiel wie dies beim bekannten Stand der Technik durch Geräte realisiert wird, die zum Beispiel am Gürtel oder in der Tasche des Patienten fixiert bzw. getragen werden.

Am oder im Applikator kann ein Elektrolytreservoir zur Elektrodenbenetzung vorgesehen sein. Der Patient erhält also hierdurch die Möglichkeit bei beginnender Austrocknung der Elektroden diese selbst leicht anzufeuchten. Wahlweise kann auch die Zufuhr des Elektrolyts über kommunizierende Röhren mit den Elektrodenhaltern von einem geeigneten Sensor automatisch gesteuert werden.

In einer Ausgestaltung der Erfindung besteht der Applikator und/oder Halterungen zur Elektrodenaufnahme aus einem verformbaren Kunststoffmaterial. Hierdurch besteht die Möglichkeit, den Applikator individuell an bestimmte Kopfformen, insbesondere im Stirn-, Nasen- oder Nackenbereich des Patienten anzupassen und hierdurch eine tatsächliche Individualisierung für den betreffenden Patienten vorzunehmen.

Die Halterungen und/oder die Stimulationselektroden können aus einem leitfähigen Kunststoffmaterial bestehen, so dass sich die Konstruktion des Applikators vereinfacht und über ein derartiges leitfähiges Material die Stromversorgung bzw. das Weiterleiten der Stimulationsimpulsfolgen realisierbar ist.

Es liegt im Sinne der Erfindung, dass am Applikator eine Tasche oder eine ähnliche Aufnahme für Ersatzelektroden vorhanden ist. Bei Verlust einer austauschbaren Elektrode kann die Behandlung fortgesetzt werden, was insbesondere dann von Vorteil ist, wenn der zu behandelnde Patient sich nicht in der Nähe der ihn sonst versorgenden ärztlichen Praxis befindet.

Insbesondere für stationäre oder klinische Anwendungen kann der Applikator zur Kombination mit einer an sich bekannten EEG-Elektrodenhaube kombiniert ausgeführt werden.

Wie bereits angedeutet, kann der Applikator eine Einheit zur Bestimmung des Übergangswiderstandes zwischen den Stimulationselektroden und der Hautoberfläche des Patienten besitzen, um bei abnormalen, insbesondere zu hohen Übergangswiderständen den Impulsgenerator abzuschalten bzw. den Stromfluss zu reduzieren.

Es hat sich gezeigt, dass das erfindungsgemäße System zur lokalen Aktivierung des menschlichen Auges und des Gehirns auch zur Sehleistungsertüchtigung, insbesondere zur Aktivierung von Nervenzellen im menschlichen Auge und Gehirn für solche Personen bzw. Probanden Anwendung finden kann, die unter extremen Gravitations- und/oder Umweltbedingungen zum Teil komplizierte Tätigkeiten ausüben müssen, wie dies beispielsweise bei Piloten, Raumfahrern, aber auch Tiefseetauchern der Fall ist.

Durch eine Behandlung dieser Personen vor, während und/oder nach deren Einsatz unter den geschilderten extremen Bedingungen bleibt die Sehleistung erhalten und es ist ein konzentriertes und gefahrloseres Arbeiten möglich.

In Ausgestaltung der Erfindung besteht die Möglichkeit, den vorgestellten Applikator so auszubilden bzw. anzupassen, dass dieser in eine Spezialausrüstung für die oben genannten Personen integriert werden kann. Hier geht es insbesondere um das Integrieren des Applikators in einen Piloten-, Raumfahrer- oder Tiefseetaucherhelm.

Die Erfindung soll nachstehend anhand eines Ausführungsbeispieles sowie unter Zuhilfenahme von Figuren näher erläutert werden.

Hierbei zeigt:
- Fig. 1: einen beispielhaften, am Kopf eines Probanden angebrachten Applikator in Vorderansicht; und
- Fig. 2: den Applikator in Rückansicht mit Blick auf den Hinterkopf des Probanden.

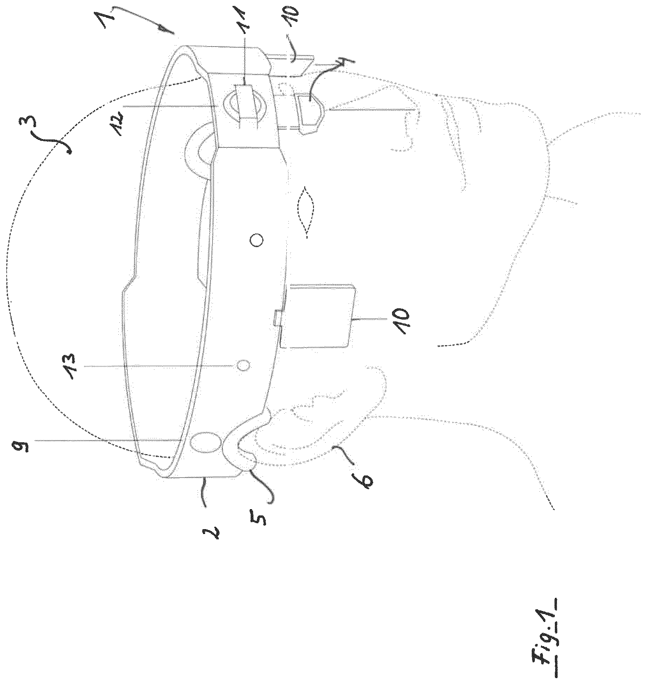

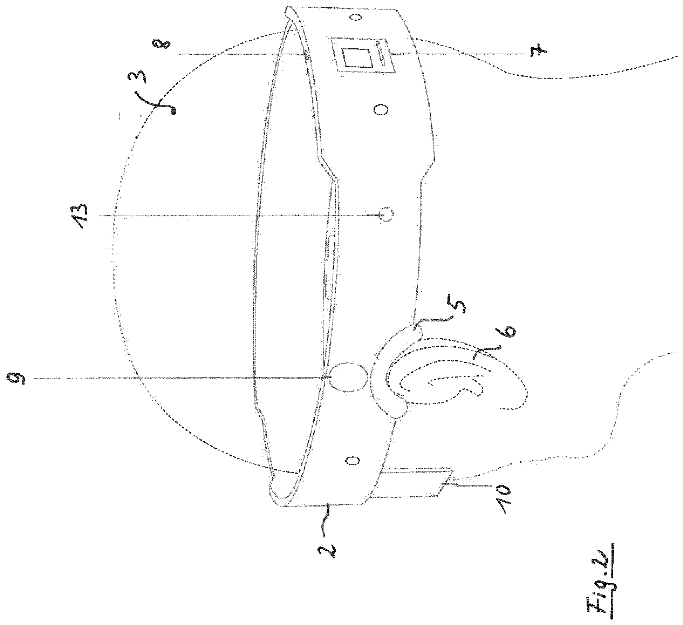

Der Applikator 1 gemäß den Figuren 1 und 2 besteht aus einem Grundkörper 2 aus einem mindestens teilweise elastischen Material und ist individuell an die Kopfform des angedeuteten Probanden 3 anpassbar.

Der Grundkörper 2 ist als am Kopf des Probanden 3 anlegbares ring- oder kronenförmiges Gebilde realisiert, wobei eine Nasenrückenabstützung 4 vorhanden ist, die zur Anpassung an anatomische Gegebenheiten eine Verstellmöglichkeit umfasst.

Weiterhin steht der Grundkörper 2 mit Ohrabstützungen 5 für das linke und rechte Ohr 6 des Probanden 3 in Verbindung.

Im Hinterkopfbereich ist der Grundkörper 2 mit einer Aufnahme für elektronische Komponenten versehen.

Hier kann es sich zum einen um eine Speichereinheit 7 handeln, welche Stimulationsprogramme umfasst. Weiterhin kann bevorzugt in diesem Hinterkopfbereich ein Impulsgenerator zur Erzeugung elektrischer Stimulationssignale nebst Steuereinheit zum Bereitstellen patientenspezifischer Stimulationssignalfolgen untergebracht oder angebracht werden.

Über eine Schnittstelle 8, zum Beispiel ausgeführt als USB-Port, kann eine Datenübertragung erfolgen, aber auch ein Laden von Sekundärzellen 9 zum Zweck der Stromversorgung der notwendigen elektrischen oder elektronischen Komponenten erfolgen.

Eine derartige Sekundärzelle 9 kann zum Beispiel eine auswechselbare Lithiumbatterie sein, die in einer diesbezüglichen Aussparung im Grundkörper 2 untergebracht ist.

Stimulationselektroden 10 sind auswechselbar am Grundkörper 2 befestigt und sind so angeordnet, dass diese rechts und links vom Auge im Bereich der Schläfe des angedeuteten Patientenkopfes auf- oder anliegen.

Im Frontbereich des Grundkörpers 2 ist eine Justiervorrichtung 12 vorhanden, die mit einem Beschriftungsfeld 11 versehen sein kann, um Raum für ein Firmenlogo, Handhabungshinweise oder aber auch für das Anbringen eines Nutzernamens oder einer Nutzerkennzeichnung zu schaffen.

In dem Grundkörper 2 können Sensoren 13 angebracht oder eingebettet sein, um eine nicht-invasive Bestimmung der Durchblutung der Haut und des Gehirns und zur Bestimmung der Sauerstoffsättigung durchzuführen. Diese Sensoren sind entfernt von den Stimulationselektroden 10 befindlich.

## Patentansprüche

1. System zur lokalen Aktivierung des menschlichen Auges und des Gehirns zum Sehleistungstraining, insbesondere zur Aktivierung von Nervenzellen im menschlichen Auge und Gehirn für eine Stärkung des Residualsehens bei bestehenden Gesichtsfelddefekten mittels nicht invasiver Wechselstromimpulsbehandlung (tACS), bestehend aus einem Applikator (1) zum Führen eines Stromflusses zum Auge und Gehirn und zur Anregung der Durchblutung und Aktivierung von Nervenzellen, insbesondere retinaler Ganglienzellen, einem Impulsgenerator zur Erzeugung von elektrischen Stimulationssignalen sowie einer Datenverarbeitungs- und Steuereinheit zum Bereitstellen patientenspezifischer Stimulationssignalfolgen, wobei der Applikator mindestens zwei Stimulationselektroden (10) aufweist, welche am Kopf des Probanden **(3)** zur Anlage bringbar sind, wobei am Applikator, die Stimulationselektroden so fixiert sind, dass diese rechts und links vom Auge im Bereich der Schläfe des Patientenkopfes auf- oder anliegen,
**dadurch gekennzeichnet, dass**
der Applikator mindestens eine Anordnung zur nicht invasiven Bestimmung der Durchblutung der Haut und des Gehirns und zur Bestimmung der Sauerstoffsättigung, auf der Basis der fNIR-Spektroskopie, aufweist, wobei der NIR-Emitter und der mindestens eine zugehörige NIR-Detektor in entfernter Position von den Stimulationselektroden am Kopf des Patienten und separat von den Stimulationselektroden positioniert ist, und die NIR-Spektroskopiedaten, in Pausen oder am Ende von Stimulationssignalfolgen, erfasst werden, um die Zeitdauer einer anhaltenden, verbesserten Durchblutung und/oder Sauerstoffsättigung zu bestimmen, so dass hiernach ein aktualisiertes Betreiben der Datenverarbeitungs- und Steuereinheit mit einer optimalen Stimulationsfolge, die zu einer maximalen Durchblutungssteigerung und zu einem am längsten anhaltendenden Nacheffekt führt, realisiert ist, derart, dass nach der Stimulation die Blutflusssteigerung und die neuronale Aktivierung möglichst lange anhält.

2. System nach Anspruch 1,
**dadurch gekennzeichnet, dass**
der Applikator als am Kopf anlegbares ring- oder kronenförmiges Gebilde ausgeführt ist, wobei eine Nasenrückenabstützung und/oder eine Ohrabstützung und/oder eine Hinterkopfabstützung und im Hinterkopfbereich eine Aufnahme für elektronische Komponenten und zum Gewichtsausgleich vorgesehen sind.

3. System nach Anspruch 1 oder 2,
**dadurch gekennzeichnet, dass**
die Stimulationselektroden als Trocken- oder Nass-Kissenelektrode ausgebildet sind.

4. System nach einem der vorangegangenen Ansprüche,
**dadurch gekennzeichnet, dass**
am Applikator Fixierpunkte zum Anbringen, insbesondere Anclipsen, weiterer Elektroden vorhanden sind, welche eine Wechselstrombeaufschlagung auch im Bereich unterhalb der Augen des Patienten ermöglichen.

5. System nach einem der vorangegangenen Ansprüche,
**dadurch gekennzeichnet, dass**
am oder im Applikator eine Schnittstelle zur drahtgebundenen oder drahtlosen Datenübertragung ausgebildet ist.

6. System nach einem der vorangegangenen Ansprüche,
**dadurch gekennzeichnet, dass**
am oder im Applikator ein Feuchtereservoir zur Elektrodenbenetzung vorhanden ist.

7. System nach einem der vorangegangenen Ansprüche,
**dadurch gekennzeichnet, dass**
der Applikator und/oder Halterungen zur Elektrodenaufnahme aus einem verformbaren Kunststoffmaterial bestehen.

8. System nach Anspruch 10,
**dadurch gekennzeichnet, dass**
die Halterungen und/oder die Stimulationselektroden aus einem leitfähigen Kunststoffmaterial bestehen.

9. System nach einem der vorangegangenen Ansprüche,
**dadurch gekennzeichnet, dass**
am Applikator eine Tasche zur Aufnahme von Ersatzelektroden vorhanden ist.

10. System nach einem der vorangegangenen Ansprüche,
**dadurch gekennzeichnet, dass**
eine Einheit zur Bestimmung des Übergangswiderstandes zwischen den Stimulationselektroden und der Hautoberfläche des Patienten ausgebildet ist, um bei abnormalen, insbesondere zu hohen Widerständen den Impulsgenerator abzuschalten.

11. System nach einem der vorangegangenen Ansprüche,
**dadurch gekennzeichnet, dass**
die Position der Stimulationselektroden einer F7-F8 Anordnung gemäß der Nomenklatur des EEG 10-20 Systems entspricht.

## Claims

1. System for local activation of the human eye and brain for visual performance training, in particular for activating nerve cells in the human eye and brain to strengthen residual vision in existing visual field defects by means of non-invasive alternating current pulse treatment (tACS), consisting of an applicator (1) for conducting a current flow to the eye and brain and for stimulating blood circulation and activating nerve cells, in particular retinal ganglion cells, a pulse generator for generating electrical stimulation signals, and a data processing and control unit for providing patient-specific stimulation signal sequences, wherein the applicator has at least two stimulation electrodes (10) that can be applied to the head of the test subject (3), wherein the stimulation electrodes are fixed to the applicator in such a way that they rest on or are applied to the right and left sides of the eye in the temple area of the patient's head,
**characterized in that**
the applicator has at least one arrangement for non-invasive determination the blood flow in the skin and brain and for determining the oxygen saturation based on fNIR spectroscopy, wherein the NIR emitter and the at least one associated NIR detector are positioned at a distance from the stimulation electrodes on the patient's head and separately from the stimulation electrodes, and the NIR spectroscopy data is recorded during pauses or at the end of stimulation signal sequences in order to determine the duration of sustained, improved blood flow and/or oxygen saturation, so that thereafter, updated operation of the data processing and control unit is realized with an optimal stimulation sequence that leads to a maximum increase in blood flow and the longest lasting aftereffect, such that after stimulation, the increase in blood flow and neuronal activation lasts as long as possible.

2. System according to claim 1,
**characterized in that**
the applicator is designed as a ring- or crown-shaped structure that can be placed on the head, with a bridge support and/or an ear support and/or a back-of-the-head support and a receptacle for electronic components and for weight balancing provided in the back-of-the-head area.

3. System according to claim 1 or 2,
**characterized in that**
the stimulation electrodes are designed as dry or wet pad electrodes.

4. System according to one of the preceding claims,
**characterized in that**
fixing points are provided on the applicator for attaching, in particular clipping on, further electrodes, which enable alternating current to be applied also in the area below the patient's eyes.

5. System according to one of the preceding claims,
**characterized in that**
an interface for wired or wireless data transmission is formed on or in the applicator.

6. System according to one of the preceding claims,
**characterized in that**
a moisture reservoir for electrode wetting is provided on or in the applicator.

7. System according to one of the preceding claims,
**characterized in that**
the applicator and/or holders for receiving electrodes are made of a deformable plastic material.

8. System according to claim 10,
**characterized in that**
the holders and/or the stimulation electrodes are made of a conductive plastic material.

9. System according to one of the preceding claims,
**characterized in that**
a pocket for holding replacement electrodes is provided on the applicator.

10. System according to one of the preceding claims,
**characterized in that**
a unit for determining the contact resistance between the stimulation electrodes and the patient's skin surface is designed to switch off the pulse generator in the event of abnormal, in particular excessively high, resistance.

11. System according to one of the preceding claims,
**characterized in that**
the position of the stimulation electrodes corresponds to an F7-F8 arrangement according to the nomenclature of the EEG 10-20 system.

## Revendications

1. Système d'activation locale de l'œil humain et du cerveau pour l'entraînement des performances visuelles, en particulier pour l'activation des cellules nerveuses dans l'œil humain et le cerveau afin de renforcer la vision résiduelle en cas de défauts du champ visuel existants au moyen d'un traitement non invasif par impulsions de courant alternatif (tACS), composé d'un applicateur (1) pour conduire un flux de courant vers l'œil et le cerveau et pour stimuler la circulation sanguine et l'activation des cellules nerveuses, en particulier des cellules ganglionnaires rétiniennes, d'un générateur d'impulsions pour générer des signaux de stimulation électriques, ainsi que d'une unité de traitement et de commande des données pour fournir des séquences de signaux de stimulation spécifiques au patient, l'applicateur comportant au moins deux électrodes de stimulation (10) pouvant être appliquées sur la tête du sujet (3), les électrodes de stimulation étant fixées sur l'applicateur de manière à être placées ou à reposer à droite et à gauche de l'œil, dans la région de la tempe de la tête du patient,
**caractérisé en ce que**
l'applicateur comporte au moins un dispositif pour la détermination non invasive de la circulation sanguine dans la peau et le cerveau et pour la détermination de la saturation en oxygène, sur la base de la spectroscopie fNIR, l'émetteur NIR et le au moins un détecteur NIR associé étant positionnés à distance des électrodes de stimulation sur la tête du patient et séparément des électrodes de stimulation, et les données de spectroscopie NIR sont enregistrées pendant les pauses ou à la fin des séquences de signaux de stimulation afin de déterminer la durée d'une circulation sanguine et/ou d'une saturation en oxygène améliorées et durables, de sorte qu'ensuite, un fonctionnement actualisé de l'unité de traitement et de commande des données est réalisé avec une séquence de stimulation optimale qui conduit à une augmentation maximale de la circulation sanguine et à un effet résiduel le plus durable possible, de telle sorte qu'après la stimulation, l'augmentation du flux sanguin et l'activation neuronale persistent aussi longtemps que possible.

2. Système selon la revendication 1,
**caractérisé en ce que**
l'applicateur est conçu comme une structure en forme d'anneau ou de couronne pouvant être placée sur la tête, avec un support pour l'arête nasale et/ou un support pour les oreilles et/ou un support pour l'arrière de la tête et, dans la zone arrière de la tête, un logement pour les composants électroniques et pour l'équilibrage du poids.

3. Système selon la revendication 1 ou 2,
**caractérisé en ce que**
les électrodes de stimulation sont conçues comme des électrodes à coussin sec ou humide.

4. Système selon l'une des revendications précédentes,
**caractérisé en ce que**
l'applicateur comporte des points de fixation pour la mise en place, en particulier le clipsage, d'électrodes supplémentaires, qui permettent d'appliquer un courant alternatif également dans la zone située sous les yeux du patient.

5. Système selon l'une des revendications précédentes,
**caractérisé en ce qu'**
une interface pour la transmission de données filaire ou sans fil est formée sur ou dans l'applicateur.

6. Système selon l'une des revendications précédentes,
**caractérisé en ce qu'**
un réservoir d'humidité destiné à humidifier les électrodes est prévu sur ou dans l'applicateur.

7. Système selon l'une des revendications précédentes,
**caractérisé en ce que**
l'applicateur et/ou les supports destinés à recevoir les électrodes sont constitués d'une matière plastique déformable.

8. Système selon la revendication 10,
**caractérisé en ce que**
les supports et/ou les électrodes de stimulation sont constitués d'un matériau plastique conducteur.

9. Système selon l'une des revendications précédentes,
**caractérisé en ce qu'**
un compartiment destiné à recevoir des électrodes de rechange est prévu sur l'applicateur.

10. Système selon l'une des revendications précédentes,
**caractérisé en ce qu'**
une unité est conçue pour déterminer la résistance de contact entre les électrodes de stimulation et la surface cutanée du patient afin de désactiver le générateur d'impulsions en cas de résistances anormales, en particulier trop élevées.

11. Système selon l'une des revendications précédentes,
**caractérisé en ce que**
la position des électrodes de stimulation d'un agencement F7-F8 correspond à la nomenclature du système EEG 10-20.
